(19) Europäisches Patentamt
European Patent Office
Office européen des brevets

(11) Publication number: **0 086 438**
**A1**

# (12) EUROPEAN PATENT APPLICATION

(21) Application number: **83101163.0**

(22) Date of filing: **08.02.83**

(51) Int. Cl.³: **C 07 D 237/32,** C 07 D 307/88

(30) Priority: **13.02.82 JP 20685/82**

(43) Date of publication of application: **24.08.83**
**Bulletin 83/34**

(84) Designated Contracting States: **CH DE FR GB IT LI**

(71) Applicant: **Nippon Kayaku Kabushiki Kaisha, New Kaijo Bldg. 2-1, Marunouchi 1-chome Chiyoda-ku, Tokyo 100 (JP)**

(72) Inventor: **Nishizawa, Rinzo, 3-18-14, Sugamo Toshima-ku, Tokyo (JP)**
Inventor: **Mashiba, Akihiro, 14-34, Hon-cho, Kazo-shi Saitama-ken (JP)**
Inventor: **Yoshida, Masao, 2-5-18 A-611, Daimon-cho, Higashikurume-shi Tokyo (JP)**
Inventor: **Takei, Yukio, 7-3-5, Namiki, Abiko-shi Chiba-ken (JP)**

(74) Representative: **Türk, Dietmar, Dr. rer. nat. et al, Redies, Redies, Türk & Gille Patentanwälte Brucknerstrasse 20, D-4000 Düsseldorf 13 (DE)**

(54) **Process and intermediates for preparing 4-hydroxymethyl-1-phthalazone derivatives.**

(57) The present invention relates to a novel process and intermediates for preparing 4-hydroxymethyl-1-phthalazone derivatives represented by the following reaction scheme:

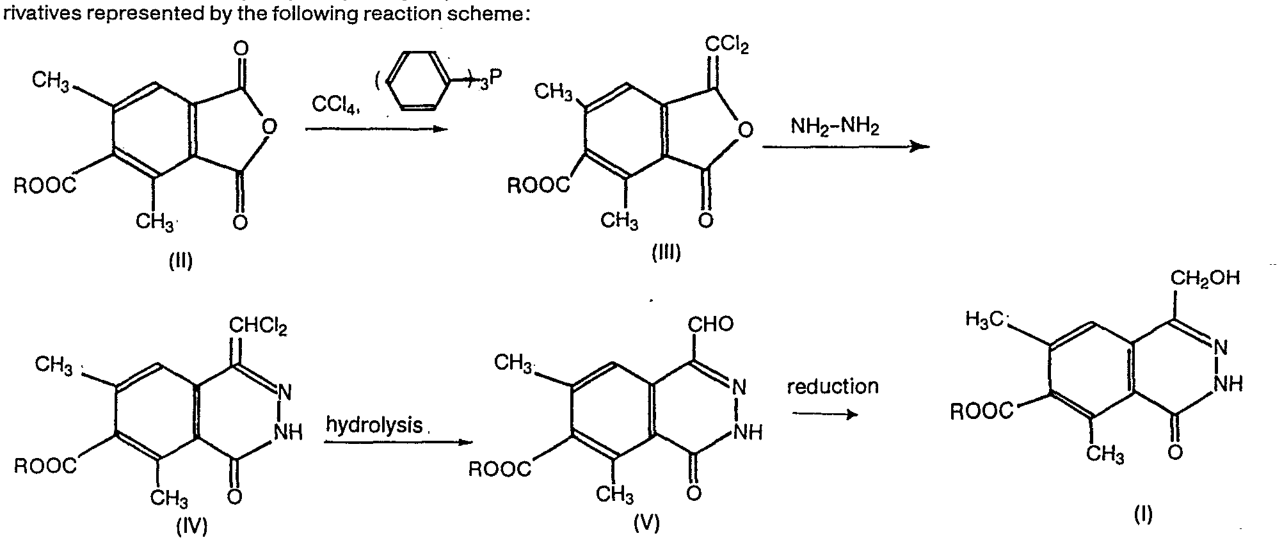

(II) (III) (IV) (V) (I)

EP 0 086 438 A1

ACTORUM AG

SUMMARY OF THE INVENTION

This invention relates to a process for preparing 4-hydroxymethyl-1-phthalazone derivatives represented by the general formula (I):

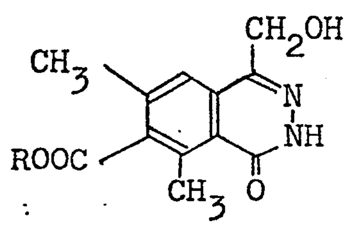

(I)

wherein R is a lower alkyl group having 1 to 6 carbon atoms,

and intermediates therefor. More specifically, the invention relates to a process for preparing 4-hydroxymethyl-1-phthalazone derivatives represented by the above formula (I) which comprises the steps of:

(a) reacting carbon tetrachloride and triphenylphosphine with a 4-alkoxycarbonyl-3,5-dimethylphthalic anhydride (hereinafter referred to as phthalic anhydride derivative) represented by the general formula:

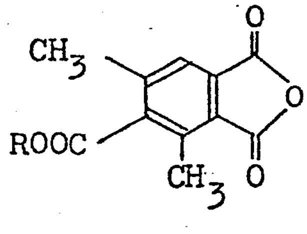

(II)

wherein R is as defined above,

to produce 6-alkoxycarbonyl-3-dichloromethylene-5,7-dimethylphthalide (hereinafter referred to as 3-dichloromethylenephthalide derivative) represented by the general formula:

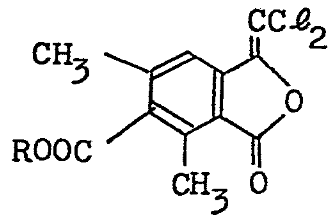

(III)

wherein R is as defined above,

(b) reacting a hydrazine with the compound (III) obtained in the above step (a) to produce 7-alkoxycarbonyl-4-dichloromethyl-6,8-dimethyl-1-phthalazone (hereinafter referred to as 4-dichloromethyl-1-phthalazone derivative) represented by the general formula:

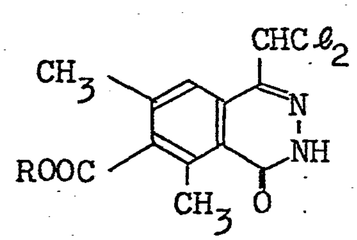

(IV)

wherein R is as defined above,

(c) and hydrolyzing the above compound (IV) to form 7-alkoxycarbonyl-6,8-dimethyl-4-formyl-1-phthalazone represented by the general formula (V):

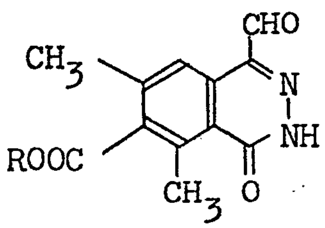

(V)

wherein R is as defined above,

and reducing the above compound (V).

The invention also relates to intermediates therefor, that is, compounds represented by the general formulae (III) and (IV).

Among compounds of the general formula (I) obtainable according to the process of this invention, the one having an ethyl group for R in said formula (I) is generally called phthalazinol. This compound has pharmacological activities such as cyclic AMP phosphodiesterase inhibiting action and platelet aggregation inhibiting action and its medicinal application for the treatment of sensory disturbance caused by cerebral stroke or central pain is anticipated.

The 3-dichloromethylenephthalide derivatives of the general formula (III) and the 4-dichloromethyl-1-phthalazone derivatives of the general formula (IV) obtained according to this invention are novel compounds not found in the literature.

As a result of extensive researches for a more advantageous preparation process for phthalazinol, the

present inventors had previously developed a method using as starting materials a compound of formula (II) and a triphenylphosphorane derivative (U.S. Patent Specification No. 4,350,813).

Further studies have led the present inventors to the findings that a 3-dichloromethylenephthalide derivative of formula (III) can be obtained in a high yield, with substantially no by-production of a positional isomer, i.e., 5-alkoxycarbonyl-4,6-dimethylphthalide represented by the general formula:

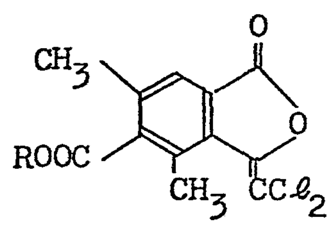

wherein R is as defined above,

by reacting a phthalic anhydride derivative of formula (II) with triphenylphosphine in carbon tetrachloride; that a reaction of said 3-dichloromethylenephthalide derivative with a hydrazine can easily afford a 4-dichloromethylphthalazone derivative of formula (IV) in a high yield; that this 4-dichloromethylphthalazone derivative, when hydrolyzed, is easily converted into a 4-formyl-1-phthalazone derivative of formula (V); and that this 4-formyl-1-phthalazone derivative can be

reduced to give a 4-hydroxymethyl-1-phthalazone derivative of the general formula (I).

The present invention was attained on the basis of these findings.

According to the process of this invention, it is possible to obtain objective compounds of formula (I) from compounds of formula (II) in four steps and all the starting materials necessary for the process are easily available, so that this process is an excellent industrial method for the preparation of said objective compounds.

The 4-formyl-1-phthalazone derivatives represented by formula (V) have been hitherto synthesized by oxidizing 4-hydroxymethyl-1-phthalazone derivatives represented by formula (I) (U.S. Patent Specification No. 4,182,876), so that they were of no value at all as an intermediate in the preparation of the compounds of formula (I) including phthalazinol. The present invention has afforded them a usefulness as an intermediate for the first time.

DETAILED DESCRIPTION OF THE INVENTION

The process of this invention is a sequence of reactions represented by the following scheme:

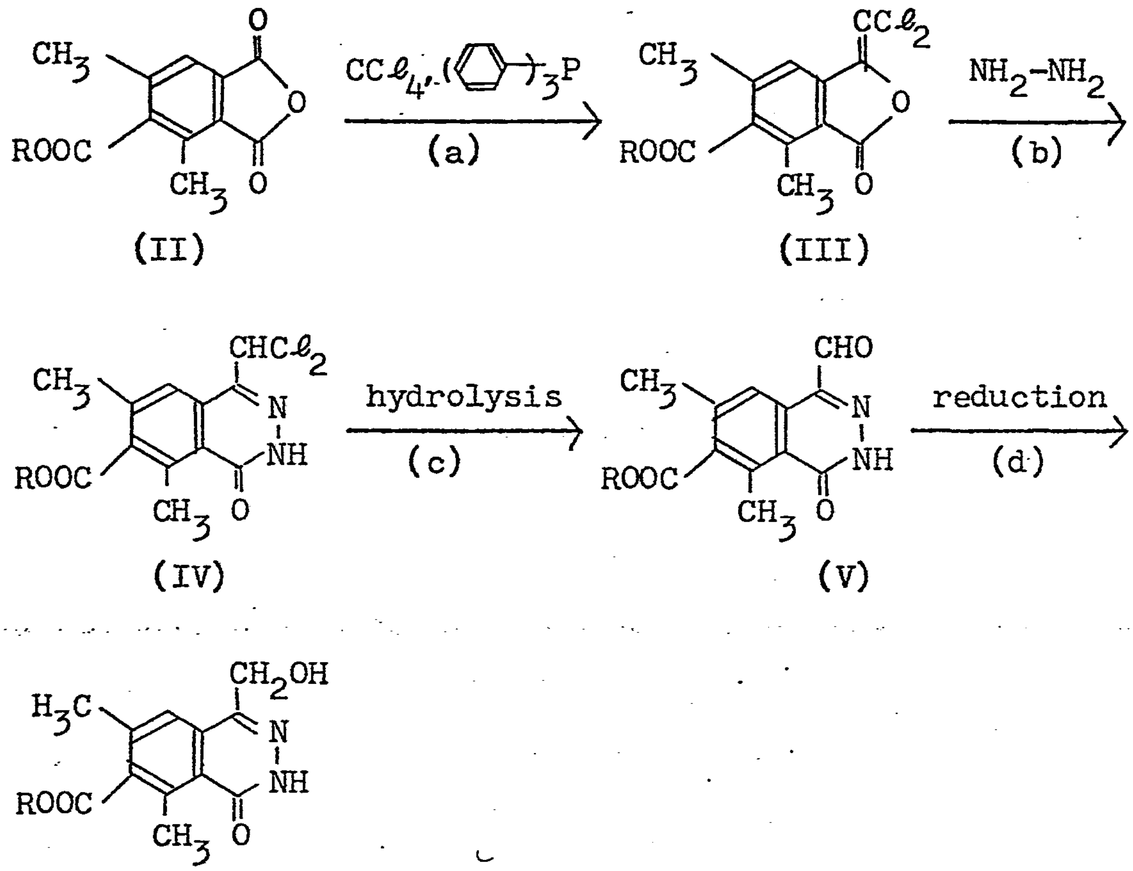


(II) (III) (IV) (V) (I)

where R's in formulae (I) to (V) are each an alkyl group having 1 to 6 carbon atoms such as methyl, ethyl, n-propyl, isopropyl, n-butyl, isobutyl, n-pentyl, isopentyl, n-hexyl, isohexyl and the like.

The step (a) in the process of this invention is accomplished by reacting triphenylphosphine with a phthalic anhydride derivative of formula (II) in carbon tetrachloride or in a solvent containing the same.

As typical examples of the phthalic anhydride

derivatives of formula (II) used as a starting material in step (a), the following may be cited:

4-ethoxycarbonyl-3,5-dimethylphthalic anhydride

4-methoxycarbonyl-3,5-dimethylphthalic anhydride

4-n-propoxycarbonyl-3,5-dimethylphthalic anhydride

4-n-butoxycarbonyl-3,5-dimethylphthalic anhydride

As the solvent used for the reaction in step (a), it is most desirable to use carbon tetrachloride which serves as a reactant at the same time, but one may also use an organic solvent which takes no part in the reaction. Among the organic solvents usable for this purpose are aromatic hydrocarbons such as xylene, toluene, or benzene, ethers such as diethyl ether, diisopropyl ether, tetrahydrofuran, or dioxane, esters such as ethyl acetate, butyl acetate, or methyl acetate, halogenated hydrocarbons such as chloroform, dichloroethane, trichloroethane, tetrachloroethane, or trichloroethylene, amides such as dimethylformamide, or dimethylacetamide, and dimethyl sulfoxide.

The amount of triphenylphosphine used in step (a) is not subject to any specific restrictions except that it should not be less than 2 equivalents to the phthalic anhydride derivative of formula (II), but it is desirable that triphenylphosphine is used in an amount of 2 to 4 equivalents to said phthalic anhydride derivative.

The amount of carbon tetrachloride may be also arbitrarily selected provided that it is not less than one equivalent to the compound of formula (II), but it is desirable that the amount of carbon tetrachloride used is at least 5 equivalents, more preferably 5 to 100 equivalents to the compound of formula (II).

The reaction can be accomplished at a temperature between room temperature and the boiling point of a solvent used, but it is preferred to perform the reaction at a temperature from 50°C to the boiling point of the solvent used. The reaction is usually completed in 3 to 24 hours.

To isolate a 3-dichloromethylenephthalide derivative of formula (III) from the reaction mixture, the mixture is concentrated under reduced pressure and the residue is dissolved in a small quantity of a mixed solvent consisting of benzene and hexane. The obtained solution is passed through a silica gel column and eluted with a benzene-hexane mixed solvent. The fraction containing the objective substance is collected and concentrated under reduced pressure.

The step (b) in the process of this invention is performed by reacting a hydrazine with a 3-dichloromethylenephthalide derivative of formula (III) obtained in step (a) in a solvent.

Examples of compounds of formula (III) used as a starting material in this step (b) are those mentioned above. As for the hydrazine used as another starting material, no specific restrictions are imposed to this reactant and it is possible to use either hydrazine or a hydrazine hydrate or a hydrous hydrazine hydrate.

The solvent used in this step (b) is also not subject to any particular restrictions provided that it is an inert solvent. The following substances may be used as the solvent in step (b): aromatic hydrocarbons such as benzene, toluene, or xylene, halogenated hydrocarbons such as chloroform, carbon tetrachloride, dichloroethane, trichloroethane, tetrachloroethane, or trichloroethylene, ethers such as diisopropyl ether, tetrahydrofuran, or dioxane, esters such as ethyl acetate or n-butyl acetate, ketones such as acetone, ethyl methyl ketone, or isobutyl methyl ketone, amides such as dimethylformamide or dimethylacetamide, dimethyl sulfoxide, acetonitrile and the like.

The amount of the hydrazine used in this step may be arbitrarily selected provided that it is at least one equivalent to 3-dichloromethylenephthalide derivative of formula (III), but usually it is desirable that the hydrazine is used in an amount of 2 to 10 equivalents.

The reaction can be conducted at a temperature between room temperature and the boiling point of a solvent used, but usually it is preferred to conduct the reaction around the boiling point of a solvent by using a solvent having a boiling point of 80°C or higher. The reaction is usually completed in 3 to 24 hours.

A 4-dichloromethyl-1-phthalazone derivative of formula (IV) may be isolated from the reaction mixture by concentrating the mixture under reduced pressure, dissolving the residue in a small quantity of a mixed solvent of benzene and ethyl acetate, passing the mixed solution through a silica gel column, eluting it with a benzene-ethyl acetate mixed solvent, collecting a fraction containing the objective substance and concentrating the fraction under reduced pressure.

Typical examples of the 4-dichloromethyl-1-phthalazone derivatives of formula (IV) obtainable in the manner described above are:

7-ethoxycarbonyl-4-dichloromethyl-6,8-dimethyl-1-phthalazone

7-methoxycarbonyl-4-dichloromethyl-6,8-dimethyl-1-phthalazone

7-n-propoxycarbonyl-4-dichloromethyl-6,8-dimethyl-1-phthalazone

7-n-butoxycarbonyl-4-dichloromethyl-6,8-dimethyl-1-phthalazone.

The hydrolysis constituting step (c) of this invention is accomplished by reacting an acid or a base with the 4-dichloromethyl-1-phthalazone derivative of formula (IV) obtained in step (b) in a solvent.

Examples of compounds (IV) used as a starting material for step (c) are those mentioned above.

The acid used in this step may be either an inorganic or an organic acid. Examples of the inorganic acids usable in this step are hydrochloric acid, sulfuric acid, nitric acid, phosphoric acid and the like, and examples of the organic acids are lower fatty acids such as formic acid, acetic acid, or propionic acid, and sulfonic acids such as benzenesulfonic acid, toluene sulfonic acid, methanesulfonic acid, or ethanesulfonic acid, and the like.

As for the base used in this step, it may be also either an inorganic or an organic base. Exemplary of the inorganic bases usable are hydroxides, carbonates or hydrogen carbonates of sodium and potassium, oxides, hydroxides and carbonates of calcium, and the like, and examples of the organic bases are triethylamine, pyridine, N-methylmorpholine, alkali metal salts of lower fatty acids such as sodium formate, sodium

acetate, potassium acetate, or sodium propionate, and the like.

The solvent used in this step may be of any type provided that the compounds of formula (IV) are soluble therein if slightly, but it is desirable to use water or an organic solvent or a mixture thereof. Any type of organic solvent may be used provided that it is miscible with water. For instance, one may use alcohols such as methanol, ethanol, propanol, isopropanol or n-butanol, ketones such as acetone, methyl ethyl ketone or isobutyl methyl ketone, ethers such as tetrahydrofuran or dioxane, amides such as dimethylformamide or dimethylacetamide, dimethyl sulfoxide, acetonitrile, lower fatty acids such as formic acid, acetic acid or propionic acid, pyridine, and the like.

When an alkali metal salt of a fatty acid is used as a hydrolyzing agent and a fatty acid as a solvent, part of the 4-dichloromethyl-1-phthalazone derivative of formula (IV) used as a starting material is converted into a 4-(diacyloxy)methyl-7-alkoxycarbonyl-6,8-dimethyl-1-phthalazone represented by the general formula:

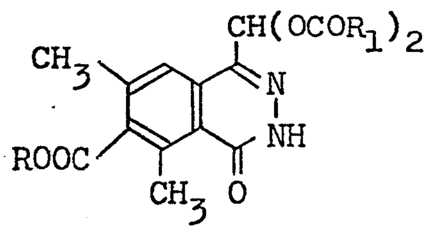

wherein $R_1$ is a hydrogen atom or a lower alkyl group, and R is as defined above,

and this compound is in turn converted into a 4-formyl-1-phthalazone derivative of formula (V) in the course of the hydrolysis.

The amount of the acid used is not specifically defined provided that it is at least 0.1 equivalent to the compound of formula (IV), but preferably it is 1 to 100 equivalents to said compound.

The base is also not specified in its amount provided that it is at least 2 equivalents to the compound of formula (IV). It is however desirable that the base is used in an amount of 2 to 100 equivalents to the compound of formula (IV).

The reaction can be accomplished at a temperature between room temperature and the boiling point of a solvent used, and it is usually completed in 30 minutes to 24 hours.

Since usually crystals of the objective substance are precipitated in the reaction mixture, the isolation

of the compound of formula (V) from the mixture can be easily effectuated by filtering out the crystals.

The 4-formyl-1-phthalazone derivatives of formula (V) which can be obtained in the manner described above include the following:

7-ethoxycarbonyl-6,8-dimethyl-4-formyl-1-phthalazone

7-methoxycarbonyl-6,8-dimethyl-4-formyl-1-phthalazone

7-n-propoxycarbonyl-6,8-dimethyl-4-formyl-1-phthalazone

7-n-butoxycarbonyl-6,8-dimethyl-4-formyl-1-phthalazone

The step (d), or the reduction, in the process of this invention can be accomplished by employing a known method for converting an aldehyde into an alcohol, for example, catalytic reduction by use of a metal catalyst, reduction by a metal hydride or reduction by an acid and a metal.

The catalytic reduction by use of a metal catalyst can be conducted in a solvent in the presence of a metal catalyst in a hydrogen atmosphere under normal pressure or under pressure. Any type of metal catalyst may be used as far as it can reduce the formyl group to a hydroxymethyl group, but preferred catalysts are palladiums such as palladium black, palladium carbon, palladium/barium sulfate, or palladium/calcium carbonate, various types of active Raney nickels, platinum or its

compounds such as platinum dioxide, rhodium and its derivatives. The amount of the catalyst to be used is not specifically defined, but if is preferred that the amount is 0.01 to 100 % based on the weight of the material to be reduced.

The solvent used in this reduction step may be arbitrarily selected provided that such solvent takes no part in the reaction and is capable of dissolving the material to be reduced. The preferred solvents are lower alcohols such as methanol, ethanol, n-propanol or isopropanol, ethers such as tetrahydrofuran, dioxane or bis(2-methoxyethoxy)ether (diglyme), esters such as methyl acetate, ethyl acetate or n-butyl acetate, carboxylic acids such as acetic acid or propionic acid, amides such as dimethylformamide or dimethylacetamide, dimethyl sulfoxide and the like, or a mixture of any of said solvents with water.

The reduction temperature is not specified but preferably selected from the range of 0 to 100°C. The reaction is usually completed in one to 24 hours.

The isolation of a 4-hydroxymethyl-1-phthalazone derivative represented by the general formula (I) from the reaction mixture can be easily accomplished by filtering off the catalyst, concentrating the filtrate under reduced pressure and, if necessary, recrystallizing the product from a suitable solvent, for example, 50 % aqueous

acetone.

The reduction by use of a metal hydride can be attained by the action of a metal hydride on a compound of formula (V) in a solvent. No specific restrictions are imposed on the metal hydride to be used for the reduction, but alkali metal salts or alkaline earth metal salts of boron hydride are preferred and, among them, sodium borohydride is particularly preferred. The amount of the metal hydride to be used for the reduction may be arbitrarily selected provided that it is not less than the theoretical amount for the substance to be reduced, but usually it is desirable that the amount is 1.2 to 2 times the theoretical.

As for the solvent used for this reduction, it is possible to employ all the solvents cited as usable in the catalytic reduction except for the carboxylic acids. The reaction temperature may be arbitrarily selected between 0°C and the boiling point of a solvent used, and the reaction is completed in usually 1 to 24 hours. The isolation of a 4-hydroxymethyl-1-phthalazone of formula (I) from the reaction mixture can be easily accomplished by concentrating the mixture under reduced pressure, adding dilute hydrochloric acid to the residue, filtering out precipitated crystals and, if necessary, recrystallizing them with a suitable solvent such as 50 %

aqueous acetone.

The reduction by use of an acid and a metal can be accomplished by adding a metal powder in a solvent containing an acid. Both inorganic and organic acids may be used for this reduction. Preferred examples of the inorganic acids are hydrochloric acid, sulfuric acid and the like, and preferred examples of the organic acids are acetic acid, propionic acid, benzenesulfonic acid, toluenesulfonic acid, methanesulfonic acid, ethanesulfonic acid and the like. The acid amount needs be enough for maintaining the acidity of the reaction solution but is not specifically defined otherwise. Any metal that is capable of producing hydrogen upon reaction with the acid may be used, but zinc, iron, tin and the like are preferred. The amount of the metal to be used for the reduction may be arbitrarily selected above the theoretical amount, but it is desirable that the amount is 2 to 100 times the theoretical for bringing the reaction to completion. As for the solvent used for this reduction, all the solvents cited as usable in the catalytic reduction may be employed.

The reaction temperature may be arbitrarily selected preferably between $0^{o}C$ and the boiling point of a solvent used, and the reaction is completed usually in 1 to

24 hours. The isolation of a 4-hydroxymethyl-1-phthalazone of formula (I) from the reaction mixture can be accomplished in the same way as in the case of reduction using a metal catalyst.

The phthalic anhydride derivatives of formula (II) usable as a starting material in this invention are known compounds (see Japanese Patent Laid-Open No. 27153/1980) and can be easily synthesized from ethyl isodehydroacetate and dichloromaleic anhydride.

The present invention will now be described in further detail by way of the examples thereof.

Example 1

20 mℓ of carbon tetrachloride was added to 1.00 g (4.03 mmol) of 4-ethoxycarbonyl-3,5-dimethylphthalic anhydride and 3.38 g (12.1 mmol) of triphenylphosphine and the mixture was heated under reflux for 15 hours. The reaction mixture was concentrated under reduced pressure and carbon tetrachloride was distilled off to obtain a crude product. This crude product was subjected to column chromatography by using 70 g of silica gel and a hexane-benzene mixed solvent as a developing solvent, and the fractions containing the objective substance were collected and concentrated under reduced pressure. Thus, 1.11 g of 3-dichloromethylene-6-ethoxycarbonyl-5,7-dimethylphthalide was obtained (yield: 87 %).

Mp: 115-116$^{o}$C.

IR spectrum: $\nu_{max}^{KBr} cm^{-1}$ = 2980, 1760, 1725, 1600, 1280, 1185, 1150, 1020, 940.

NMR spectrum ($CDCl_3$):

$\delta$ = 1.42 (3H, t, J = 7 Hz, $OCH_2\underline{CH_3}$), 2.48,
2.67 (3H, 3H, s, s, phenyl-$CH_3$X2),
4.46 (2H, q, J = 7 Hz, $O\underline{CH_2}CH_3$),
7.82 (1H, s, phenyl-H).

MS spectrum: m/e = 315 ($M^+$).

Example 2

1.24 g (8.06 mmol) of carbon tetrachloride and 10 mℓ of toluene were added to 1.00 g (4.03 mmol) of 4-ethoxycarbonyl-3,5-dimethylphthalic anhydride and 2.64g (10.1 mmol) of triphenylphosphine and the mixture was heated under reflux for 15 hours. The reaction solution was then treated in the same manner as in Example 1 to obtain 1.01 g of 3-dichloromethylene-6-ethoxycarbonyl-5,7-dimethylphthalide (yield: 85 %). This product completely agreed with that of Example 1 in melting point, IR spectrum, NMR spectrum and MS spectrum.

Example 3

3.10 g (20.2 mmol) of carbon tetrachloride and 10 mℓ of dioxane were added to 1.00 g (4.03 mmol) of 4-ethoxycarbonyl-3,5-dimethylphthalic anhydride and

2.32 g ( 8.87 mmol ) of triphenylphosphine and the mixture was heated under reflux for 15 hours and then treated in the same way as in Example 1 to obtain 1.05 g of 3-dichloromethylene-6-ethoxycarbonyl-5,7-dimethylphthalide (yield: 83 %). This substance completely agreed with the product of Example 1 in melting point, IR spectrum, NMR spectrum and MS spectrum.

Example 4

3.28 g (21.4 mmol) of carbon tetrachloride and 10 mℓ of chloroform were added to 1.00 g (4.27 mmol) of 4-methoxycarbonyl-3,5-dimethylphthalic anhydride and 2.79 g (10.68 mmol) of triphenylphosphine and the mixture was heated under reflux for 15 hours. The same treatment as in Example 1 afforded 1.14 g of 3-dichloromethylene-6-methoxycarbonyl-5,7-dimethylphthalide (yield: 89 %).

Mp: 137-139°C

IR spectrum:

$\nu_{max}^{KBr}$ $cm^{-1}$ = 1770, 1725, 1600, 1285, 1170, 1010, 945.

NMR spectrum ($CDCl_3$):

δ = 2.46, 2.63 (3H, 3H, s, s, phenyl-$CH_3$X2), 3.97 (3H, s, $OCH_3$), 7.77 (1H, s, phenyl-H).

MS spectrum: m/e = 301 ($M^+$).

Example 5

200 mg (0.64 mmol) of 3-dichloromethylene-6-ethoxycarbonyl-5,7-dimethylphthalide was dissolved in 20 mℓ of xylene and 80 mg (1.3 mmol) of an 80 % hydrazine hydrate solution was added thereto. The resulting solution was heated under reflux for 12 hours and then concentrated under reduced pressure and xylene was distilled off to obtain a crude product. This crude product was subjected to column chromatography by using 20 g of silica gel and a benzene-ethyl acetate mixed solvent as a developing solvent and the fractions containing the objective substance were collected and concentrated under reduced pressure to obtain 173 mg of 4-dichloromethyl-7-ethoxycarbonyl-6,8-dimethyl-1-phthalazone (yield: 82 % ).

Mp: 187-189°C.

IR spectrum:

$$\nu_{max}^{KBr} cm^{-1} = 3150, 2920, 1740, 1660, 1596, 1270, 1156, 1119, 1035, 745.$$

NMR spectrum ($CDC\ell_3$):

δ = 1.41 (3H, t, J = 7 Hz, $OCH_2\underline{CH_3}$), 2.52, 2.90 (3H, 3H, s, s, phenyl-$CH_3$X2), 4.47 (2H, q, J = 7 Hz, $O\underline{CH_2}CH_3$), 6.83 (1H, s, $C\underline{H}C\ell_2$), 8.13 (1H, s, phenyl-H), 11.04 (1H, s, NH).

MS spectrum: m/e = 329 ($M^+$).

Example 6

200 mg (0.64 mmol) of 3-dichloromethylene-6-

ethoxycarbonyl-5,7-dimethylphthalide was dissolved in 20 mℓ of n-propanol, followed by addition of 80 mg (1.3 mmol) of an 80 % hydrazine hydrate solution and heating under reflux for 20 hours. The reaction solution was then treated in the same way as in Example 5 to obtain 168 mg of 4-dichloromethyl-7-ethoxycarbonyl-6,8-dimethyl-1-phthalazone (yield: 80 %). This substance completely agreed with the product of Example 5 in melting point, IR spectrum, NMR spectrum and MS spectrum.

Example 7

200 mg (0.64 mmol) of 3-dichloromethylene-6-ethoxycarbonyl-5,7-dimethylphthalide was dissolved in 20 mℓ of dioxane, to which was then added 200 mg (3.2 mmol) of an 80 % hydrazine hydrate solution and the mixed solution was heated under reflux for 20 hours. The reaction solution was then treated according to the process of Example 5 to obtain 164 mg of 4-dichloromethyl-6-ethoxycarbonyl-6,8-dimethyl-1-phthalazone (yield: 78 %). This substance showed a complete agreement with that obtained in Example 5 in melting point, IR spectrum, NMR spectrum and MS spectrum.

Example 8

200 mg (0.66 mmol) of 3-dichloromethylene-6-methoxycarbonyl-5,7-dimethylphthalide was dissolved in

20 m$\ell$ of xylene, to which was then added 83 mg (1.32 mmol) of an 80 % hydrazine hydrate solution and the mixed solution was heated under reflux for 12 hours. The resulting reaction solution was subjected to the same treatment as in Example 5 to obtain 175 mg of 4-dichloromethyl-7-methoxycarbonyl-6,8-dimethyl-1-phthalazone (yield: 83 %).

Mp: 226-228$^{o}$C.

IR spectrum:

$\nu_{max}^{KBr}$ $cm^{-1}$ = 3150, 3000, 1730, 1655, 1600, 1280, 1235, 1150, 1110, 1030, 730.

NMR spectrum ($CDC\ell_3$):

$\delta$ = 2.50, 2.88 (3H, 3H, s, s, phenyl-$CH_3$X2), 4.0 (3H, s, $OCH_3$), 6.85 (1H, s, $CHC\ell_2$), 8.15 (1H, s, phenyl-H), 10.73 (1H, s, NH).

MS spectrum: m/e = 315 ($M^+$).

Example 9

To 500 mg (1.52 mmol) of 4-dichloromethyl-7-ethoxycarbonyl-6,8-dimethyl-1-phthalazone were added 20 m$\ell$ of acetic acid and 20 m$\ell$ of water and the mixture was heated under reflux for 2 hours. Upon completion of the reaction, the reaction solution was cooled and precipitated crystals were filtered out, washed with water and dried to obtain 400 mg of 4-formyl-7-ethoxycarbonyl-6,8-dimethyl-1-phthalazone

(yield: 96 %).

Mp: 219-223°C.

IR spectrum:

$\nu_{max}^{KBr}$ cm$^{-1}$ = 2900, 1725, 1700, 1655, 1590, 1260, 1220, 1110.

NMR spectrum ($CDCl_3$):

δ = 1.43 (3H, t, J = 7 Hz, $OCH_2C\underline{H_3}$), 2.53, 2.90 (3H, 3H, s, s, phenyl-$CH_3$X2), 4.48 (2H, q, J = 7 Hz, $O\underline{CH_2}CH_3$), 8.83 (1H, s, phenyl-H), 9.87 (1H, s, CHO), 10.7 (1H, s, NH).

MS spectrum: m/e 274 ($M^+$).

Example 10

8.0 mℓ of methanol and 2.0 mℓ of 1N hydrochloric acid were added to 100 mg (0.304 mmol) of 4-dichloromethyl-7-ethoxycarbonyl-6,8-dimethyl-1-phthalazone and the mixture was heated under reflux for 24 hours. The reaction solution was concentrated under reduced pressure and the solvent was distilled off to obtain a crude product. This crude product was subjected to column chromatography using 10 g of silica gel and a benzene-ethyl acetate mixed solvent as a developing solvent, and the fractions containing the objective substance were collected and concentrated under reduced pressure to obtain 41.7 mg of 4-formyl-7-ethoxycarbonyl-6,8-dimethyl-1-phthalazone (yield: 50 %). This compound completely agreed with that obtained in Example 9 in melting point, IR spectrum, NMR spectrum and MS spectrum.

Example 11

8.0 mℓ of methanol and 2 mℓ of an aqueous solution of 1N sodium hydroxide were added to 100 mg (0.304 mmol) of 4-dichloromethyl-7-ethoxycarbonyl-6,8-dimethyl-1-phthalazone and the mixture was heated under reflux for 15 hours. The reaction solution was treated in the same manner as in Example 10 to obtain 29.2 mg of 4-formyl-7-ethoxycarbonyl-6,8-dimethyl-1-phthalazone (yield: 35 %). This compound was in complete agreement with that obtained in Example 9 in melting point, IR spectrum, NMR spectrum and MS spectrum.

Example 12

4 mℓ of acetic acid and 4 mℓ of water were added to 100 mg (0.317 mmol) of 4-dichloromethyl-7-methoxycarbonyl-6,8-dimethyl-1-phthalazone and the mixture was heated under reflux for 2 hours. The same treatment as in Example 9 afforded 78.4 mg of 4-formyl-7-methoxycarbonyl-6,8-dimethyl-1-phthalazone (yield: 95 %).

Mp: 237-241°C.

IR spectrum:

$\nu_{max}^{KBr}$ $cm^{-1}$ = 3400, 3000, 2920, 1710, 1660, 1590, 1270, 1225, 1110, 1030, 820.

NMR spectrum ($CDCl_3$):

δ = 2.48, 2.85 (3H, 3H, s, s, phenyl-$CH_3$x2), 3.98 (3H, s, $OCH_3$), 8.88 (1H, s, phenyl-H), 9.88 (1H, s, CHO), 10.7 (1H, s, NH).

MR spectrum: m/e = 260 ($M^+$).

Example 13

250 mg (3.04 mmol) of sodium acetate and 10 mℓ of acetic acid were added to 100 mg (0.304 mmol) of 4-dichloromethyl-7-ethoxycarbonyl-6,8-dimethyl-1-phthalazone and the mixture was heated under reflux for 2 hours. 10 mℓ of water was added to this reaction solution and precipitated crystals were filtered out, washed with water and dried to obtain 80 mg of 4-formyl-7-ethoxycarbonyl-6,8-dimethyl-1-phthalazone (yield: 96 %). This compound completely coincided with that obtained in Example 9 in melting point, IR spectrum, NMR spectrum and MS spectrum.

Example 14

206 mg (3.04 mmol) of sodium formate and 10 mℓ of formic acid were added to 100 mg (0.304 mmol) of 4-dichloromethyl-7-ethoxycarbonyl-6,8-dimethyl-1-phthalazone and the mixture was stirred at room temperature for 3 hours. After the reaction, the

reaction solution was treated according to Example 13 to obtain 80 mg of 4-formyl-7-ethoxycarbonyl-6,8-dimethyl-1-phthalazone (yield: 96 %). This compound was in complete accord with that obtained in Example 9 in melting point, IR spectrum, NMR spectrum and MS spectrum.

Example 15

292 mg (3.04 mmol) of sodium propionate and 10 mℓ of propionic acid were added to 100 mg (0.304 mmol) of 4-dichloromethyl-7-ethoxycarbonyl-6,8-dimethyl-1-phthalazone and the mixture was stirred at room temperature for 3 hours. After the reaction was completed, the reaction solution was treated in the same manner as in Example 13 to obtain 78 mg of 4-formyl-7-ethoxycarbonyl-6,8-dimethyl-1-phthalazone (yield: 94 %). This compound completely agreed with that obtained in Example 9 in melting point, IR spectrum, NMR spectrum and MS spectrum.

Example 16

250 mg (3.04 mmol) of sodium acetate and 10 mℓ of dimethylformamide were added to 100 mg (0.304 mmol) of 4-dichloromethyl-7-ethoxycarbonyl-6,8-dimethyl-1-phthalazone and the mixture was stirred at room temperature for 2 hours. After the reaction, the reaction solution was concentrated under reduced pressure and the

solvent was distilled off. 20 mℓ of water was added to the residue and stirred for 30 minutes and precipitated crystals were filtered out, washed with water and dried to obtain 76 mg of 4-formyl-7-ethoxycarbonyl-6,8-dimethyl-1-phthalazone (yield: 91 %). This compound completely agreed with that obtained in Example 9 in melting point, IR spectrum, NMR spectrum and MS spectrum.

Example 17

The process of Example 16 was repeated by using 10 mℓ of tetrahydrofuran instead of dimethylformamide and heating the mixture under reflux for 2 hours, followed by otherwise the same treatment as in Example 16 to obtain 75 mg of 4-formyl-7-ethoxycarbonyl-6,8-dimethyl-1-phthalazone (yield: 90 %). This compound completely agreed with that obtained in Example 9 in melting point, IR spectrum, NMR spectrum and MS spectrum.

Example 18

The process of Example 16 was repeated by using 60 mg (0.79 mmol) of propionic acid and 10 mℓ of pyridine instead of 250 mg of sodium acetate and 10 mℓ of dimethylformamide to obtain 75 mg of 4-formyl-7-ethoxycarbonyl-6,8-dimethyl-1-phthalazone (yield: 90 %). This compound agreed completely with that obtained in Example 9 in melting point, IR spectrum, NMR spectrum

and MS spectrum.

Example 19

100 mg (0.365 mmol) of 4-formyl-7-ethoxycarbonyl-6,8-dimethyl-1-phthalazone was suspended in 20 mℓ of methanol, to which was then added 27.6 mg (0.730 mmol) of sodium borohydride under ice cooling and the mixture was stirred at room temperature for 2 hours. After the reaction, the reaction solution was concentrated under reduced pressure and methanol was distilled off. The residue was dissolved in 20 mℓ of water and the pH was adjusted to 3.5-5.0 with acetic acid. Precipitated crystals were filtered out, washed with water and dried to obtain 96.2 mg of 4-hydroxymethyl-7-ethoxycarbonyl-6,8-dimethyl-1-phthalazone (yield: 95 %). Mp: 171-173°C. This compound completely agreed with a separately synthesized standard preparation in IR spectrum and NMR spectrum.

4-Hydroxymethyl-7-methoxycarbonyl-6,8-dimethyl-1-phthalazone was likewise synthesized from 4-formyl-7-methoxycarbonyl-6,8-dimethyl-1-phthalazone. It had a melting point of 202-203°C and completely agreed with a separately synthesized standard preparation in IR spectrum and NMR spectrum.

WHAT IS CLAIMED IS:

1. A process for preparing 4-hydroxymethyl-1-phthalazone derivatives represented by the general formula:

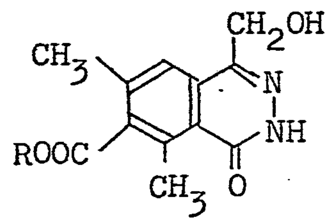

wherein R is an alkyl group having 1 to 6 carbon atoms,

which comprises the steps of:

(a) reacting carbon tetrachloride and triphenylphosphine with a 4-alkoxycarbonyl-3,5-dimethylphthalic anhydride represented by the general formula:

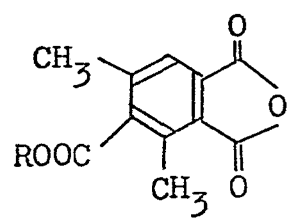

wherein R is as defined above,

to produce a corresponding 6-alkoxycarbonyl-3-dichloromethylene-5,7-dimethylphthalide represented by the general formula:

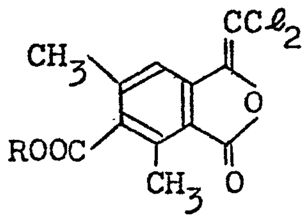

wherein R is as defined above,

(b) reacting this compound with a hydrazine to produce a corresponding 7-alkoxycarbonyl-4-dichloromethyl-6,8-dimethyl-1-phthalazone represented by the general formula:

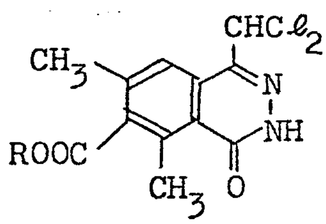

wherein R is as defined above,

(c) hydrolyzing this compound in the presence of an acid or a base to form a corresponding 7-alkoxycarbonyl-6,8-dimethyl-4-formyl-1-phthalazone represented by the general formula:

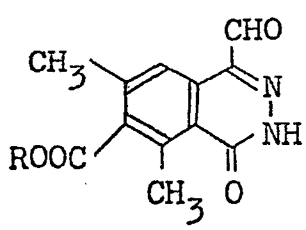

wherein R is as defined above, and

(d) reducing this compound.

2. The process according to Claim 1, wherein R in the general formulae is a methyl or ethyl group.

3. The process according to Claim 1 or 2, wherein the amount of carbon tetrachloride and that of triphenylphosphine used in step (a) are at least 1 equivalent and at least 2 equivalents, respectively, to the 4-alkoxycarbonyl-3,5-dimethylphthalic anhydride, and the amount of the hydrazine used in step (b) is at least 1 equivalent to the 6-alkoxycarbonyl-3-dichloromethylene-5,7-dimethylphthalide, and the amount of the acid or base used in step (c) is at least 0.1 equivalent in the case of the acid and at least 2 equivalents in the case of the base to the 7-alkoxycarbonyl-4-dichloromethyl-6,8-dimethyl-1-phthalazone.

4. The process according to Claim 1 or 2, wherein the amount of carbon tetrachloride and that of triphenylphosphine used in step (a) are at least 5 equivalents and 2 to 4 equivalents, respectively, to the 4-alkoxycarbonyl-3,5-dimethylphthalic anhydride, and the amount of the hydrazine used in step (b) is 2 to 10 equivalents to the 6-alkoxycarbonyl-3-dichloromethylene-5,7-dimethylphthalide, and the amount of the acid or base used in step (c) is 1 to 100 equivalents in the case of the acid and 2 to 100 equivalents in the case of the

base to the 7-alkoxycarbonyl-4-dichloromethyl-6,8-dimethyl-1-phthalazone.

5. The process according to any of Claims 1 to 4, wherein the reaction is conducted at a temperature within the range from room temperature to the boiling point of the solvent in any of steps (a) to (c).

6. The process according to any of Claims 1 to 5, wherein the reaction is conducted at a temperature from 50°C to the boiling point of the solvent in step (a) and from 80°C to the boiling point of the solvent in step (b).

7. A process for preparing the 4-hydroxymethyl-1-phthalazone derivative represented by the formula:

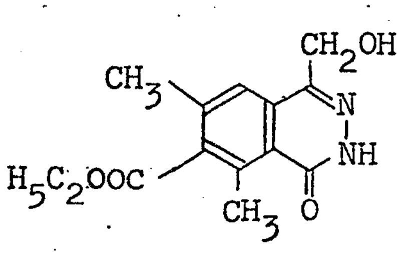

which comprises the steps of:

(a) reacting at least 5 equivalents of carbon tetrachloride and 2 to 4 equivalents of triphenylphosphine with 4-ethoxycarbonyl-3,5-dimethylphthalic anhydride represented by the formula:

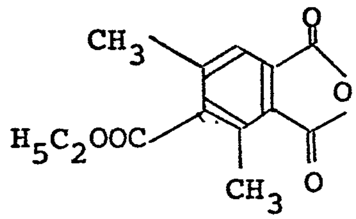

at a temperature between 50°C and the boiling point of the solvent used to produce 6-ethoxycarbonyl-3-dichloromethylene-5,7-dimethylphthalide represented by the formula:

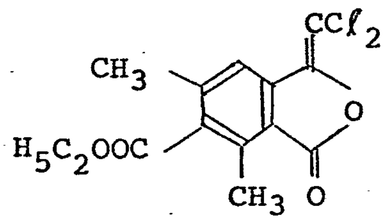

(b) reacting 2 to 10 equivalents of a hydrazine with this compound at a temperature between 80°C and the boiling point of the solvent used to produce 7-ethoxycarbonyl-4-dichloromethyl-6,8-dimethyl-1-phthalazone represented by the formula:

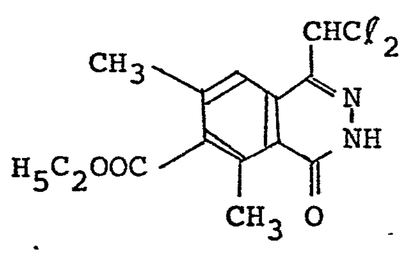

(c) hydrolyzing this compound by using 1 to 100

equivalents of an acid or 2 to 100 equivalents of a base at a temperature between room temperature and the boiling point of the solvent used to form 7-ethoxycarbonyl-6,8-dimethyl-4-formyl-1-phthalazone represented by the formula:

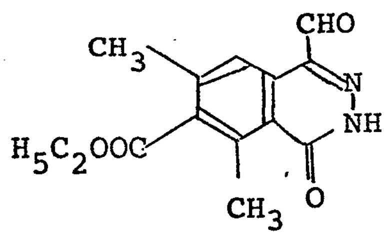

and,

(d) reducing this compound.

8. 6-Alkoxycarbonyl-3-dichloromethylene-5,7-dimethylphthalide represented by the general formula:

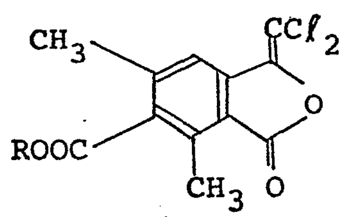

wherein R is an alkyl group having 1 to 6 carbon atoms.

9. 7-Alkoxycarbonyl-4-dichloromethyl-6,8-dimethyl-1-phthalazone represented by the general formula:

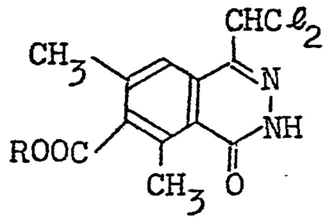

wherein R is an alkyl group having 1 to 6 carbon atoms.

0086438

European Patent Office

# EUROPEAN SEARCH REPORT

Application number

EP 83 10 1163

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | GB-A-2 055 825 (NIPPON KAYAKU)<br>* Pages 1-7 *<br>----- | 1,2,5-7 | C 07 D 237/32<br>C 07 D 307/88 |
| | | | TECHNICAL FIELDS SEARCHED (Int. Cl. 3) |
| | | | C 07 D 237/00<br>C 07 D 307/00 |

The present search report has been drawn up for all claims

| Place of search | Date of completion of the search | Examiner |
|---|---|---|
| THE HAGUE | 25-05-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82